# EUROPEAN PATENT SPECIFICATION

(11) **EP 3 335 605 B1**
(45) Date of publication and mention of the grant of the patent: **01.05.2019**
(21) Application number: 17206687.0
(22) Date of filing: 12.12.2017
(51) Int. Cl.: A47K 10/38

(54) **ROLL CARRIER FOR A PAPER SHEET WITH CONTROLLED DISTRIBUTION**
ROLLENTRÄGER FÜR EINEN PAPIERBOGEN MIT KONTROLLIERTER VERTEILUNG
SUPPORT DE ROULEAU POUR UNE FEUILLE DE PAPIER AVEC RÉPARTITION CONTRÔLÉE

(30) Priority: 14.12.2016 ES 201631464 U
(43) Date of publication of application: 20.06.2018
(73) Proprietor: Sempere Borja, Carlos, 03802 Alcoy (Alicante) (ES)
(72) Inventor: Sempere Borja, Carlos, 03802 Alcoy (Alicante) (ES)
(74) Representative: Ungria López, Javier

(56) References cited:
- EP-A1- 2 394 551
- EP-A2- 1 938 725
- FR-A1- 2 873 562
- US-A- 5 715 971
- US-A- 6 082 663

## Description

### OBJECT OF THE INVENTION

The present invention, as expressed in the title of this description, refers to a roll carrier for a paper sheet with controlled distribution that makes it possible to extract the paper sheet with transversal perforation lines housed inside a housing that forms part of the roll carrier that is the invention; where the paper sheet is extracted from the internal space of the housing through a through bore; and where a roll of the paper sheet is housed inside the housing. From this premise, the object of the invention is to make it easier to extract the paper sheet wrapped around the roll, preventing any interruptions during the process of extraction and helping to break the paper sheet along the transversal perforation lines thereof.

### TECHNICAL PROBLEM TO ADDRESS AND BACKGROUND OF THE INVENTION

Currently there are known paper roll carriers that generally comprise a housing defining an internal space where a roll of a paper sheet is housed in, so that said paper sheet is extracted through an opening or hole that connects the internal space of the housing with the outside, so that in order to extract the paper sheet a user must use their hand to pull one end of the paper sheet that protrudes from outside the housing of the roll carrier.

The Spanish patent under publication number ES2344212 T3 refers to a paper distributor comprising a housing where a roll of a paper sheet is housed in, which includes transversal die-cuts or perforation lines defining rectangular paper sheets; with the housing comprising a distribution hole for unrolling the paper sheet. In this invention the paper sheet is toilet paper and the distributor comprises a tubular mouthpiece with the distribution hole, so that the tubular mouthpiece and the paper roll make it possible to unroll the sheets of paper one by one and come out of the tubular mouthpiece with few creases; thus using the paper in an optimal and pleasant way.

The patent under publication number US 3973695 A (D01) describes a paper distributor comprising a housing where a roll of a paper sheet is housed in; where the paper sheet is extracted through a hole in the housing that has an elongated configuration with widened ends.

The patent under publication number FR 2761252 A1 (D02) describes a system for distributing paper comprising a housing where a roll of a paper sheet is housed in; where the paper sheet is extracted through a conical mouthpiece that protrudes from outside the housing and has a tapered end.

The patent under publication number US 5785274 A (D03) refers to a device for housing and distributing toilet paper comprising a housing where a roll of a paper sheet is housed in; where the paper sheet is extracted through a tubular mouthpiece that protrudes from outside the housing.

The patent under publication number US 643350 (D04) refers to a paper that can be divided and that is wrapped around a roll, and which has transversal die-cut lines.

In addition, it should be noted that the European patent under publication number EP-1799083_B1 is a patent document that forms part of the family of patents related to the aforementioned Spanish patent ES 2344212 T3.

The background of said European patent cited the documents mentioned above, D01, D02, D03 and D04, so that most of them already comprised basically a housing where a paper roll is housed in, and a hole through which the paper sheet is extracted. In order to do so, a user pulls said paper sheet with one hand in order to separate an end part of said paper sheet, which is separated from the rest of the paper sheet thanks to the perforation lines incorporated in the paper sheet.

US 5 715 971 A discloses a roll carrier according to the preamble of claim 1.

### DESCRIPTION OF THE INVENTION

In order to achieve the goals and address the drawbacks mentioned in the previous sections, the invention provides a roll carrier for a paper sheet with controlled distribution that comprises a housing that defines an internal space configured to house a paper roll.

The roll carrier that is the invention also comprises an independent part with a through bore located in a tubular junction that forms part of the independent part; where said tubular junction is fitted inside a through bore of the housing; and where the independent part is housed inside the internal space of the housing.

The independent part includes an arched edge and front extensions that run from said arched edge of the independent part; where said arched edge and the front extensions are resting on curved areas of the housing.

The roll carrier that is the invention comprises a base housed inside the housing, which is configured to provide support to a paper roll resting on said base.

The base comprises a curved structure that includes two opposing arched edges: first and second edge; where the first arched edge of the base includes arched extensions that fit into grooved bearing surfaces of the housing, while the second arched edge of the base includes two bridges where a series of lugs joined to the base are placed on; and wherein said lugs are coupled to a series of tabs of the independent part.

The housing may comprise a first part that includes the through hole and a second part; where the two parts of the housing are connected together by means of a hinged device located under the base; where the arched extensions of the base are fitted in the grooved bearing surfaces, and said grooved bearing surfaces are located on the second part of the housing; while the independent part is coupled to the first part of the housing.

The hinged device may comprise two groups of fins arranged alternately, which are joined to the two parts of the housing, and that are joined together by an axis fitted in opposing perforations that are located on the two groups of fins joined to the two parts of the housing.

The roll carrier that is the invention also may comprise an anchoring device located in an area opposite to the hinged device; where the anchoring device comprises an elastic part joined to the second part of the housing in combination with a protrusion joined to the first part of the housing. Said elastic part comprises a projecting end section that includes a tab; so that in the closed position of the housing, the tab of the elastic part is hooked on the protrusion of the first part of the housing.

The tab of the elastic part may feature a slant configured to contact the protrusion when the housing is closed; so that once the housing is closed, the tab of the elastic part is hooked on the protrusion of the first part of the housing.

The end section of the elastic part may be connected to a control part that is guided along a series of through slots of the first part of the housing; where said control part features a hole in which a part of the end section of the control part is fitted into when the housing is in the closed position.

In one of the embodiments of the invention, the tubular junction of the independent part comprises a truncated-cone configuration which is complemented by a truncated-cone configuration of the through hole of the housing.

In one of the embodiments of the invention, the through bore of the independent part comprises a truncated-cone configuration.

Hereinafter, in order to give a better understanding of the description, the object of the invention has been detailed in a series of drawings that are an integral part of the report and are for illustration purposes and without limitation.

### BRIEF DESCRIPTION OF THE FIGURES

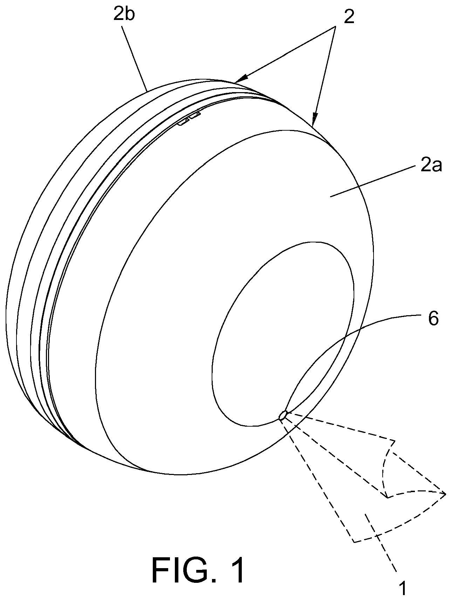

Figure 1.- It shows a perspective view of the roll carrier for a paper sheet with controlled distribution that is the object of the invention.

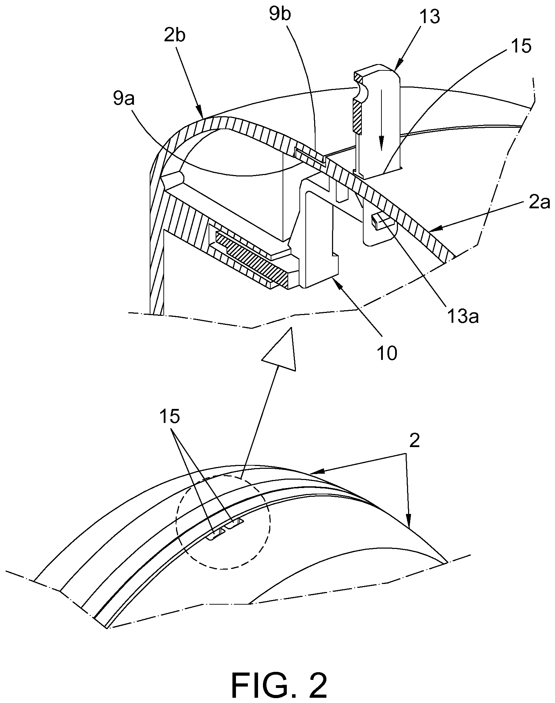

Figure 2.- It shows a view of a part of the roll carrier with a detailed expanded section.

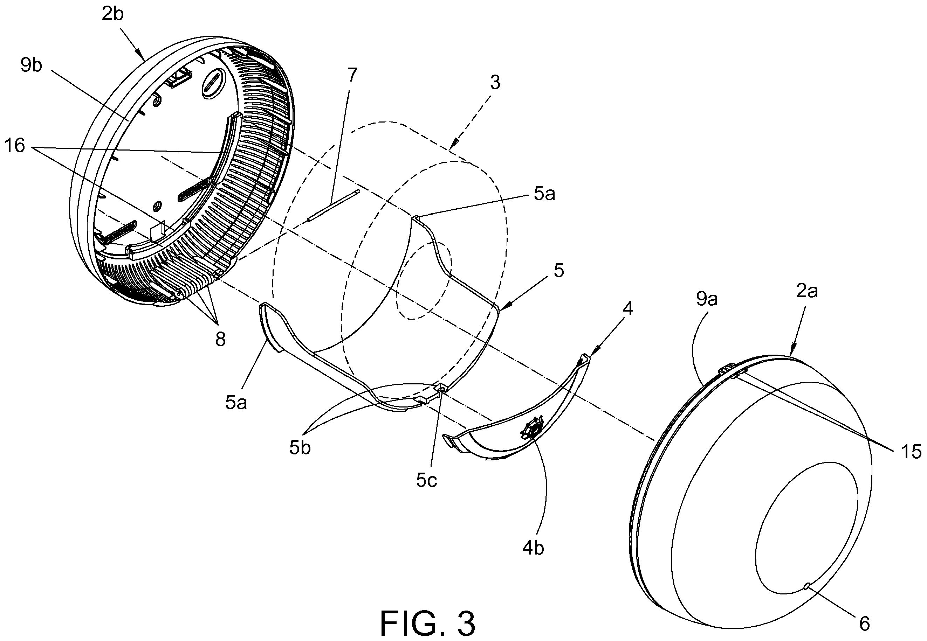

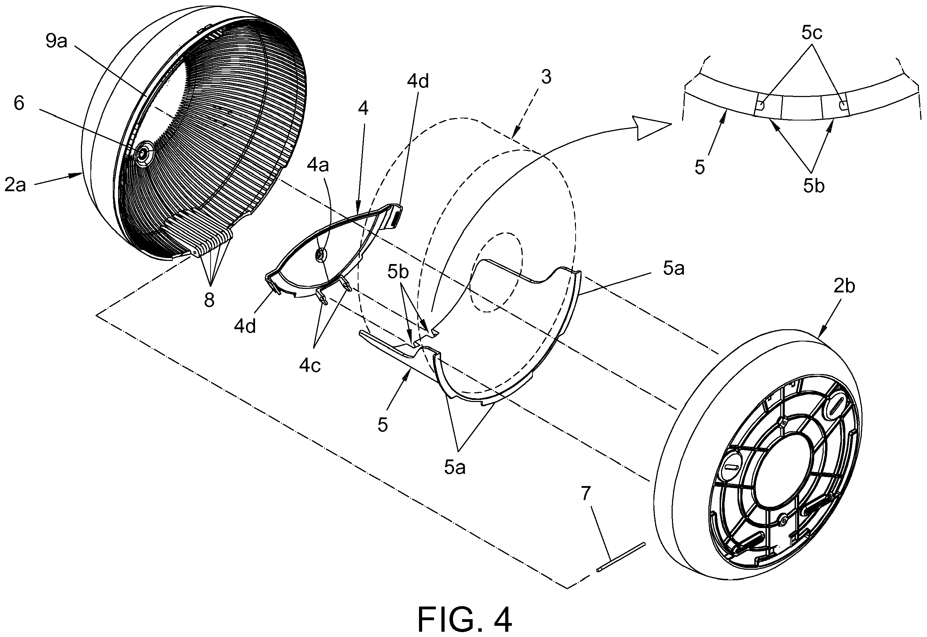

Figure 3 and 4.- They show perspective views of the roll carrier that is the invention.

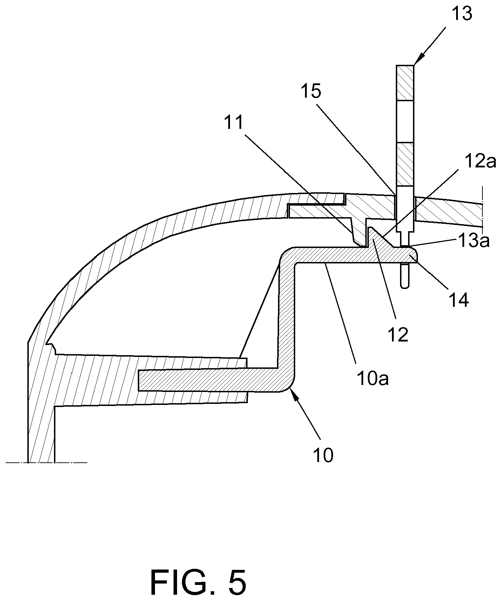

Figure 5.- It shows a cross-section view of one of the parts of the roll carrier that is the invention.

### DESCRIPTION OF A SAMPLE EMBODIMENT

Considering the numbering adopted in the figures, the roll carrier for a paper sheet 1 with controlled distribution comprises a housing 2 defining an internal space where a paper roll 3 is housed in; where the paper roll 1 is extracted from the internal space of the housing 2 through a through bore 4a with a conical configuration that is located in an independent part 4 that forms part of the paper carrier, where the independent part 4 is fixed to the housing 1 and where said independent part 4 is located in the internal space of the housing 2.

The paper sheet 1 of the roll 3 is extracted from the internal space of the housing 2 through the through bore 4a, so that a user pulls said paper sheet 1 with one hand in order to separate an end part of said paper sheet 1, which is separated from the rest of the paper sheet 1 thanks to a series of perforation lines incorporated in said paper sheet 1. Said through bore 4a is a tapered tube through which the corrugated paper sheet 1 is unrolled, offering a degree of resistance to extraction when the user pulls from the paper sheet 1 so that this resistance to extraction causes the end part of the paper sheet to tear and separate along the last perforation line joining the end part to the rest of the paper sheet 1.

The roll carrier that is the invention includes a base 5 where the paper roll 3 rests on; where the base 5 is an independent element located in the internal space of the housing 2 and where said base 5 is also fixed to the housing 1.

The independent part 4 includes a tubular junction 4b that is embedded inside a through hole 6 of the housing 2, while the independent part 4 is flat against an inner side of the housing 2, where said tubular junction 4b includes the through bore 4a through which the corrugated paper sheet 1 is extracted.

In addition, the housing 2 comprises a first part 2a that includes the through hole 6 and a second part 2b that includes means for fixing the roll carrier to a wall or to a different support.

The two parts 2a, 2b are connected together by means of a hinged device formed of two groups of fins 8 arranged alternately, which are joined to the two parts 2a, 2b of the housing 2, and that are joined together by an axis 7 fitted in a series of opposing perforations that are located on the fins 8 of the housing 2.

In the embodiment shown in the figures, the hinged device is located on a lower area under the base 5 where the paper roll 3 rests on.

The two parts 2a, 2b of the housing 2 comprise mouths connected together by means of a tongue and groove coupling in the closed position of the housing 2, so that said tongue and groove coupling is interrupted by the fins 8 of the hinged device.

Said tongue and groove coupling comprises an outer fin 9b joined to the second part 2b of the housing 2, and an inner fin 9a joined to the first part 2a of the housing 2, where the fins 9a, 9b are superimposed in the closed position of the housing 2 forming a staggered stop that ensures that the housing 2 is closed.

The roll carrier that is the invention also comprises an anchoring device to ensure the housing 2 is closed by means of an elastic hook; where in the embodiment shown in the figures said anchoring device is located in the area opposite the axis 7 of the hinged device.

The anchoring device comprises an elastic part 10 fixed to the second part 2b of the housing 2 in combination with a protrusion 11 joined to the first part 2a of the housing 2; with said elastic part 10 comprising a projecting end section 10a that includes a tab 12; so that in the closed position of the housing 2, the tab 12 of the elastic part 10 is hooked on the protrusion 11 of the first part 2a.

The tab 12 of the elastic part 10 features a slant 12a configured to contact the protrusion 11 when the housing is closed 2; so that when this contact takes place a projecting end section 10a of the elastic part 10 is deformed; and where once the housing 2 is closed, the tab 12 passes the protrusion 11, hooking said tab 12 on said protrusion 11.

In order to unlock and release the closed housing 2 in order to access the internal space thereof, it is first necessary to push down the projecting end section 10a that includes the tab 12 of the elastic part in order to release the hooked tab 12 anchored on the protrusion 11 in the closed position of the housing 2.

In order to do so, the end section 10a of the elastic part 10 is depressed by means of a control part 13 guided along through slots 15 on the first part 2a of the housing 2, where said control part 13 includes a hole 13a, so that in the closed position of the housing 2, a portion 14 of the projecting end section 10a of the elastic part 10 is fitted into the hole 13a of the control part 13.

The base 5 where the roll 3 rests on has a curved configuration that includes two opposing arched edges: first and second edge. The first arched edge includes arched extensions 5a that fit into grooved bearing surfaces 16 of the second part 2b of the housing 2, while the second arched edge of the base 2 includes two bridges 5b where a series of lugs 5c joined to the base 5 are placed on.

The independent part 4 includes a pair of tabs 4c that are coupled to the lugs 5c of the base 5; with the independent part 4 also comprising an arched edge with front extensions 4d running therefrom where the adjacent parts of the second arched edge of the base 5 rest on. In addition, the arched edge and the front extensions 4d of the independent part 4 rest on curved areas of the first part 2a of the housing 2, and said independent part 4 also rests on the through hole 6 of the housing 2 through the truncated-cone junction 4b thereof.

## Claims

1. Roll carrier for a paper sheet 1 with controlled distribution, which comprises a housing (2) defining an internal space configured to house a paper roll (3); comprising an independent part (4) with a through bore (4a) located in a tubular junction (4b) that forms part of the independent part (4); where said tubular junction (4b) is fitted inside a through bore of the housing (2); and where the independent part is housed inside the internal space of the housing (2) and includes an arched edge and front extensions (4d) that run from said arched edge of the independent part (4); where said arched edge and the front extensions (4d) are resting on curved areas of the housing (2);
the roll carrier also comprising a base (5) housed inside the housing (2) that is configured to support a paper roll (3);
**characterized in that** the base (5) comprises a curved structure that includes two opposing arched edges: first and second edge; where the first arched edge of the base (5) includes arched extensions (5a) that fit into grooved bearing surfaces (16) of the housing (2), while the second arched edge of the base (5) includes two bridges (5b) where a series of lugs (5c) joined to the base (5) are placed on; and wherein said lugs (5c) are coupled to a series of tabs (4c) of the independent part (4).

2. Roll carrier for a paper sheet 1 with controlled distribution, according to claim 1, **characterized in that** the housing (2) comprises a first part (2a) that includes the through hole (6) and a second part (2b); where the two parts (2a, 2b) of the housing (2) are connected together by means of a hinged device located under the base (5); where the arched extensions (5a) of the base (5) are fitted in the grooved bearing surfaces (16), and said grooved bearing surfaces (16) are located on the second part (2b) of the housing (2); while the independent part (4) is coupled to the first part (2a) of the housing (2).

3. Roll carrier for a paper sheet 1 with controlled distribution, according to claim 2, **characterized in that** the hinged device comprises two groups of fins (8) arranged alternately, which are joined to the two parts (2a, 2b) of the housing (2), and that are joined together by an axis (7) fitted in opposing perforations that are located on the two groups of fins (8) joined to the two parts (2a, 2b) of the housing (2).

4. Roll carrier for a paper sheet 1 with controlled distribution, as claimed in any one of the claims 2 or 3, **characterized in that** it comprises an anchoring device located in an area opposite to the hinged device; where the anchoring device comprises an elastic part (10) joined to the second part (2b) of the housing (2) in combination with a protrusion (11) joined to the first part (2a) of the housing (2); with said elastic part (10) comprising a projecting end section (10a) that includes a tab (12); so that in the closed position of the housing (2), the tab (12) of the elastic part (10) is hooked on the protrusion (11) of the first part (2a) of the housing (2).

5. Roll carrier for a paper sheet 1 with controlled distribution, according to claim 4, **characterized in that** the tab (12) of the elastic part (10) features a slant (12a) configured to contact the protrusion (11) when the housing is closed (2).

6. Roll carrier for a paper sheet 1 with controlled distribution, as claimed in any one of the claims 4 or 5, **characterized in that** the end section (10a) of the elastic part (10) is joined to a control part (13) that is guided along a series of through slots (15) of the first part (2a) of the housing (2); where said control part (13) features a hole (13a) in which a portion (14) of the end section (10a) of the elastic part (10) is fitted into when the housing (2) is in the closed position.

7. Roll carrier for a paper sheet 1 with controlled distribution, according to claim 1, **characterized in that** the tubular junction (4b) of the independent part (4) comprises a truncated-cone configuration which is complemented by a truncated-cone configuration of the through hole (6) of the housing (2).

8. Roll carrier for a paper sheet 1 with controlled distribution, according to claim 1, **characterized in that** the through bore (4a) of the independent part (4) comprises a truncated-cone configuration.

## Patentansprüche

1. Rollenträger für eine Papierbahn (1) mit gesteuerter Verteilung, der ein Gehäuse (2) umfasst, das einen Innenraum bildet, der zum Aufnehmen einer Papierrolle (3) eingerichtet ist, einen unabhängigen Teil (4) mit einer Durchgangsbohrung (4a) umfasst, die sich in einem röhrenförmigen Übergangsbereich (4b) befindet, der einen Teil des unabhängigen Teils (4) bildet, wobei der röhrenförmige Übergangsbereich (4b) in eine Durchgangsbohrung des Gehäuses (2) eingepasst ist und der unabhängige Teil im Inneren des Innenraums des Gehäuses (2) aufgenommen ist und einen bogenförmigen Rand sowie vordere Verlängerungen (4d) einschließt, die von dem bogenförmigen Rand des unabhängigen Teils (4) aus verlaufen, wobei der bogenförmige Rand und die vorderen Verlängerungen (4d) an gekrümmten Bereichen des Gehäuses (2) anliegen;
der Rollenträger des Weiteren eine Auflage (5) umfasst, die im Inneren des Gehäuses (2) aufgenommen und so eingerichtet ist, dass sie eine Papierrolle (3) trägt;
**dadurch gekennzeichnet, dass** die Auflage eine gekrümmte Struktur umfasst, die zwei einander gegenüberliegende bogenförmige Ränder, d. h. einen ersten und einen zweiten Rand, einschließt, wobei der erste bogenförmige Rand der Auflage (5) bogenförmige Verlängerungen (5a) einschließt, die in gekehlte Lagerungsflächen (16) des Gehäuses (2) passen, der zweite bogenförmige Rand der Auflage (5) zwei Brücken (5b) einschließt, auf denen eine Reihe mit der Auflage (5) verbundener Nasen (5c) positioniert sind, und wobei die Nasen (5c) mit einer Reihe von Vorsprüngen (4c) des unabhängigen Teils (4) gekoppelt sind.

2. Rollenträger für eine Papierbahn (1) mit gesteuerter Verteilung nach Anspruch 1, **dadurch gekennzeichnet, dass** das Gehäuse (2) einen ersten Teil (2a), der das Durchgangsloch (6) einschließt, sowie einen zweiten Teil (2b) umfasst, wobei die 2 Teile (2a, 2b) des Gehäuses (2) über eine Gelenkvorrichtung miteinander verbunden sind, die sich unter der Auflage (5) befindet, die bogenförmigen Verlängerungen (5a) der Auflage (5) in die gekehlten Lagerungsflächen (16) eingepasst sind und sich die gekehlten Lagerungsflächen (16) an dem zweiten Teil (2b) des Gehäuses (2) befinden, während der unabhängige Teil (4) mit dem ersten Teil (2a) des Gehäuses (2) gekoppelt ist.

3. Rollenträger für eine Papierbahn (1) mit gesteuerter Verteilung nach Anspruch 2, **dadurch gekennzeichnet, dass** die Gelenkvorrichtung zwei Gruppen von Rippen (8) umfasst, die abwechselnd angeordnet sind, die mit den zwei Teilen (2a, 2b) des Gehäuses (2) verbunden sind und die über eine Achse (7) miteinander verbunden sind, die in einander gegenüberliegende Löcher eingepasst ist, die sich an den zwei Gruppen von Rippen (8) befinden, die mit den zwei Teilen (2a, 2b) des Gehäuses (2) verbunden sind.

4. Rollenträger für eine Papierbahn (1) mit gesteuerter Verteilung nach einem der Ansprüche 2 oder 3, **dadurch gekennzeichnet, dass** er eine Befestigungsvorrichtung umfasst, die sich in einem der Gelenkvorrichtung gegenüberliegenden Bereich befindet, wobei die Befestigungsvorrichtung einen elastischen Teil (10), der mit dem zweiten Teil (2b) des Gehäuses (2) verbunden ist, in Kombination mit einem mit dem ersten Teil (2a) des Gehäuses (2) verbundenen vorstehenden Teil (11) umfasst, und der elastische Teil (10) einen vorstehenden End-Teilabschnitt (10a) umfasst, der einen Vorsprung (12) einschließt, so dass in der geschlossenen Position des Gehäuses (2) der Vorsprung (12) des elastischen Teils (10) an dem vorstehenden Teil (11) des ersten Teils (2a) des Gehäuses (2) eingehakt ist.

5. Rollenträger für eine Papierbahn (1) mit gesteuerter Verteilung nach Anspruch 4, **dadurch gekennzeichnet, dass** der Vorsprung (12) des elastischen Teils (10) eine Abschrägung (12a) aufweist, die so eingerichtet ist, dass sie mit dem vorstehenden Teil (11) in Kontakt kommt, wenn das Gehäuse (2) geschlossen ist.

6. Rollenträger für eine Papierbahn (1) mit gesteuerter Verteilung nach einem der Ansprüche 4 oder 5, **dadurch gekennzeichnet, dass** der End-Teilabschnitt (10a) des elastischen Teils (10) mit einem Steuerungs-Teil (13) verbunden ist, der an einer Reihe von Durchgangsschlitzen (15) des ersten Teils (2a) des Gehäuses (2) entlang geführt wird, wobei der Steuerungs-Teil (13) eine Öffnung (13a) aufweist, in die ein Abschnitt (14) des End-Teilabschnitts (10a) des elastischen Teils (10) eingepasst wird, wenn sich das Gehäuse (2) in der geschlossenen Position befindet.

7. Rollenträger für eine Papierbahn (1) mit gesteuerter Verteilung nach Anspruch 1, **dadurch gekennzeichnet, dass** der röhrenförmige Übergangsbereich (4b) des unabhängigen Teils (4) eine Kegelstumpf-Form umfasst, zu der eine Kegelstumpf-Form des Durchgangslochs (6) des Gehäuses (2) komplementär ist.

8. Rollenträger für eine Papierbahn (1) mit gesteuerter Verteilung nach Anspruch 1, **dadurch gekennzeichnet, dass** die Durchgangsbohrung (4a) des unabhängigen Teils (4) eine Kegelstumpf-Form umfasst.

## Revendications

1. Support de rouleau pour une feuille de papier 1 avec une distribution contrôlée, qui comprend un boîtier (2) définissant un espace interne configuré pour recevoir un rouleau de papier (3) ; comprenant une partie indépendante (4) avec un alésage traversant (4a) situé dans une jonction tubulaire (4b) qui forme une partie de la partie indépendante (4) ; où ladite partie de jonction tubulaire (4b) est ajustée à l'intérieur d'un alésage traversant du boîtier (2) ; et où la partie indépendante est reçue à l'intérieur de l'espace interne du boîtier (2) et comprend un bord arqué et des extensions avant (4d) qui s'étendent à partir dudit bord arqué de la partie indépendante (4) ; où ledit bord arqué et les extensions avant (4d) reposent sur des aires incurvées du boîtier (2) ;
le support de rouleau comprenant également une base (5) reçue à l'intérieur du boîtier (2) qui est configurée pour supporter un rouleau de papier (3) ;
**caractérisé en ce que** la base (5) comprend une structure incurvée qui comprend deux bords arqués opposés : un premier et un second bord ; où le premier bord arqué de la base (5) comprend des extensions arquées (5a) qui correspondent à l'intérieur de surfaces de support rainurées (16) du boîtier (2), tandis que le second bord arqué de la base (5) comprend deux ponts (5b) sur lesquels une série de pattes (5c) reliées à la base (5) sont placées ; et dans lequel lesdites pattes (5c) sont couplées à une série de languettes (4c) de la partie indépendante (4).

2. Support de rouleau pour une feuille de papier 1 avec une distribution contrôlée selon la revendication 1, **caractérisé en ce que** le boîtier (2) comprend une première partie (2a) qui comprend le trou traversant (6) et une seconde partie (2b) ; où les deux parties (2a, 2b) du boîtier (2) sont reliées au moyen d'un dispositif articulé situé sous la base (5) ; où les extensions arquées (5a) de la base (5) sont ajustées dans les surfaces de support rainurées (16) et lesdites surfaces de support rainurées (16) sont situées sur la seconde partie (2b) du boîtier (2) ; tandis que la partie indépendante (4) est couplée à la première partie (2a) du boîtier (2).

3. Support de rouleau pour une feuille de papier 1 avec une distribution contrôlée selon la revendication 2, **caractérisé en ce que** le dispositif articulé comprend deux groupes d'ailettes (8) disposées en alternance, qui sont reliées aux deux parties (2a, 2b) du boîtier (2), et qui sont reliées ensemble par un axe (7) ajusté dans des perforations opposées qui sont situées sur les deux groupes d'ailettes (8) reliées aux deux parties (2a, 2b) du boîtier (2).

4. Support de rouleau pour une feuille de papier 1 avec une distribution contrôlée selon l'une quelconque des revendications 2 ou 3, **caractérisé en ce qu'**il comprend un dispositif d'ancrage situé dans une aire opposée au dispositif articulé ; où le dispositif d'ancrage comprend une partie élastique (10) reliée à la seconde partie (2b) du boîtier (2) en combinaison avec une saillie (11) reliée à la première partie (2a) du boîtier (2) ; ladite partie élastique (10) comprenant une section d'extrémité saillante (10a) qui comprend une languette (12) ; de telle sorte que dans la position fermée du boîtier (2), la languette (12) de la partie élastique (10) est accrochée à la saillie (11) de la première partie (2a) du boîtier (2).

5. Support de rouleau pour une feuille de papier 1 avec une distribution contrôlée selon la revendication 4, **caractérisé en ce que** la languette (12) de la partie élastique (10) présente une inclinaison (12a) configurée pour entrer en contact avec la saillie (11) lorsque le boîtier (2) est fermé.

6. Support de rouleau pour une feuille de papier 1 avec une distribution contrôlée selon l'une quelconque des revendications 4 ou 5, **caractérisé en ce que** la section d'extrémité (10a) de la partie élastique (10) est reliée à une partie de commande (13) qui est guidée le long d'une série de fentes traversantes (15) de la première partie (2a) du boîtier (2) ; où ladite partie de commande (13) présente un trou (13a) dans lequel une partie (14) de la section d'extrémité (10a) de la partie élastique (10) est ajustée lorsque le boîtier (2) est dans la position fermée.

7. Support de rouleau pour une feuille de papier 1 avec une distribution contrôlée, selon la revendication 1, **caractérisé en ce que** la jonction tubulaire (4b) de la partie indépendante (4) comprend une configuration en cône tronqué qui est complétée par une configuration en cône tronqué du trou traversant (6) du boîtier (2).

8. Support de rouleau pour une feuille de papier 1 avec une distribution contrôlée, selon la revendication 1, **caractérisé en ce que** l'alésage traversant (4a) de la partie indépendante (4) comprend une configuration en cône tronqué.
